(19) Europäisches Patentamt / European Patent Office / Office européen des brevets

(11) **EP 2 090 267 A2**

(12) **EUROPEAN PATENT APPLICATION**

(43) Date of publication:
**19.08.2009 Bulletin 2009/34**

(51) Int Cl.:
*A61F 2/06* (2006.01)   *E21B 17/22* (2006.01)
*F16L 11/12* (2006.01)   *B29C 47/00* (2006.01)
*F16L 9/00* (2006.01)   *B01J 8/06* (2006.01)

(21) Application number: **09001557.9**

(22) Date of filing: **18.03.2004**

(84) Designated Contracting States:
**AT BE BG CH CY CZ DE DK EE ES FI FR GB GR HU IE IT LI LU MC NL PL PT RO SE SI SK TR**

(30) Priority: **18.03.2003 GB 0306179**

(62) Document number(s) of the earlier application(s) in accordance with Art. 76 EPC:
**04721592.6 / 1 611 386**

(71) Applicant: **Heliswirl Technologies Limited**
**21 Wilson Street**
**London EC2M 2TD (GB)**

(72) Inventors:
• **Caro, Colin Gerald**
**London SW7 2AZ (US)**

• **Watkins, Nicholas V.**
**London SW7 2AZ (GB)**

(74) Representative: **Piésold, Alexander James**
**Frank B. Dehn & Co.**
**St Bride's House**
**10 Salisbury Square**
**London**
**EC4Y 8JD (GB)**

Remarks:
This application was filed on 04-02-2009 as a divisional application to the application mentioned under INID code 62.

(54) **Piping**

(57) The invention relates to piping (10) comprising a portion wherein the centreline of the portion follows a substantially helical path, wherein the amplitude of the helix is less than or equal to one half of the internal diameter of the piping. When fluid flows in such piping (10), it is made to swirl. This provides a number of advantages, such as improved in-plane mixing of the fluid, enhanced uniformity of residence time, and so on. The invention also extends to various methods of making such piping (10).

FIG. 1

EP 2 090 267 A2

**Description**

The present invention relates to piping for carrying fluids.

It is already known that fluid can flow in a "swirl flow", and this flow is discussed in WO 97/28637, in the context of penstocks and draft tubes for turbines. The swirl flow is achieved by forming the penstocks or draft tubes in such a way that their centrelines curve in three dimensions.

Swirl flow has a number of advantages over conventional flow. Pressure losses (and energy losses) through turbulence can be reduced. In addition, the velocity profile of the flow across the pipe is more uniform (or blunter) than it would be with conventional flow. As a result, fluid flowing in a swirl flow tends to act as a plunger, removing sediment or debris which may have accumulated on the pipe walls, which is of particular importance in hydroelectric plant.

Pipes having similar three-dimensional curves are also discussed in WO 02/093063, where they are used in the context of production and processing plant. In such plant, it is often necessary for pipes connecting various parts of the plant to extend for some distance, and have a number of bends. Forming the bends so that they have three-dimensional curves promotes swirl flow, and leads to reduced energy losses, reduced risk of stagnation and of sedimentation.

However, these prior art documents are only concerned with using three-dimensional curves in place of the known two-dimensional curves (such as elbow bends), so as to induce swirl flow. They are not concerned with creating swirl flow in situations where a generally straight pipe would normally be used.

One possible way of making flow swirl in a straight pipe would be to form grooves or ribs along the inner surface of the pipe, which grooves or ribs curve along the pipe (much like rifling in a gun barrel). However, this has the disadvantage of increasing the wetted perimeter of the pipe, and in the case of ribs, reducing the cross-sectional area of the pipe; both grooves and ribs can lead to increased flow resistance and consequent pressure loss.

In addition, experiment has shown that unless the Reynolds number is very low, the grooves or ribs only have an effect on the flow near the wall of the pipe, and it may be necessary to provide a long pipe in order to be sure that the flow swirls across the entire width of the pipe. Swirl in the centre of the pipe is only achieved through diffusional transfer of momentum from the flow at the wall of the pipe; the grooves or ribs do not facilitate mixing between fluid near the wall of the pipe and fluid at the centre of the pipe.

According to a first aspect of the invention, there is provided piping comprising a portion wherein the centreline of the portion follows a substantially helical path, wherein the amplitude of the helix is less than or equal to one half of the internal diameter of the piping.

When fluid enters a piece of piping shaped as a helical portion in this way, swirl flow is established almost immediately. It has been found that swirl flow is established across the entire width of the pipe within a few pipe diameters of the entry. Further, the swirl flow involves considerable secondary motion and mixing of the fluid, with mass, momentum and heat transfer between the fluid at the walls of the pipe and the fluid at the centre of the pipe.

In this specification, the amplitude of the helix refers to the extent of displacement from a mean position to a lateral extreme. So, in the case of tubing having a helical centre line, the amplitude is one half of the full lateral width of the helical centre line. The dross-sectional area of the tubing is substantially constant along its length.

In piping according to the first aspect of the invention, there is a "line of sight" along the lumen of the piping. This is distinct from a corkscrew configuration, where the helix is effectively wound around a core (either solid, or "virtual" with a core of air). It has been found that the flow at the line of sight generally has a swirl component, even though it could potentially follow a straight path.

For the purposes of this specification, the term "relative amplitude" of helical piping is defined as the amplitude divided by the internal diameter. Since the amplitude of the helical piping is less than or equal to one half of the internal diameter of the tubing, this means that the relative amplitude is less than or equal to 0.5. Relative amplitudes less than or equal to 0.45, 0.40, 0.35, 0.30, 0.25, 0.20, 0.15, 0.1 or 0.05 may be preferred. Smaller relative amplitudes provide a better use of available lateral space, in that the piping is not much wider overall than a normal straight pipe with the same cross-sectional area. Smaller relative amplitudes also result in a wider "line of sight", providing more space for the insertion of pressure gauges or other equipment along the piping. With higher Reynolds numbers, smaller relative amplitudes may be used whilst swirl flow is induced to a satisfactory extent. This will generally mean that, for a given internal diameter, where there is a high flow rate a low relative amplitude can be used whilst still being sufficient to induce swirl flow.

The angle of the helix is also a relevant factor in balancing space considerations with the desirability of having a large cross-sectional area available for flow. The helix angle is preferably less than or equal to 65°, more preferably less than or equal to 55°, 45°, 35°, 25°, 20°, 15°, 10° or 5°. As with relative amplitudes, the helix angle may be optimized according to the conditions, and in particular the viscosity, density and velocity of the fluid being carried by the piping.

Generally speaking, for higher Reynolds numbers the helix angle may be smaller whilst satisfactory swirl flow is achieved, whilst with lower Reynolds numbers a higher helix angle will be required to produce satisfactory swirl. The use of higher helix angles for faster flows (with higher Reynolds numbers) will generally be undesirable, as there may be near wall pockets of stagnant fluid. Therefore, for a given Reynolds number (or range of Reynolds numbers), the helix angle will preferably be chosen to be as low as possible to produce satisfactory swirl. In certain embodiments, the helix angle is less than 20°.

In general, the piping will have a plurality of turns of the helix. Repeated turns of the helix along the piping will tend to ensure that the swirl flow is fully developed.

Lengths of piping will normally be made with substantially the same relative amplitude and helix angle along their length; however, one or both of them may vary. Further, the helical portion may extend along the entire length of the piping, or may only extend along part of it, to "condition" the flow and to simplify connection of the piping to other pipes.

The piping may extend generally linearly (ie the axis of helical rotation may be a straight line). However, the axis may be curved, to produce a generally curved pipe. The curve of the axis may be two-dimensional or three-dimensional; if it is three-dimensional, then it is important to ensure that the swirl created by the three-dimensional curve augments the swirl created by the helical piping.

According to a second aspect of the present invention, there is provided a method of making piping comprising a portion wherein the centreline of the portion follows a substantially helical path, said method including the steps of positioning a straight flexible tubing portion adjacent to a further straight flexible member, twisting the flexible tubing portion and the flexible member around each other, and treating the flexible tubing portion so that it retains its shape.

It has been found that a flexible tubing portion, when twisted together with a further flexible member in this way, takes the form of a helical portion as described above. The relative amplitude of the helical portion can be varied by varying the diameters of the tubing portion and the member, and the pitch can be varied by varying the angle through which the ends of the assembly of the portion and the member are twisted relative to each other.

Preferably, the flexible tubing portion is prevented from kinking or otherwise deforming in an undesirable manner during twisting, and in a preferred embodiment a snugly fitting coiled spring is inserted into the tubing portion before twisting.

The flexible tubing portion can be treated to retain its shape in a number of ways. For example, it could be formed from a material which is initially flexible but sets solid over time. However, in a preferred form, it is formed from a material which can be made to retain its shape by suitable treatment (such as a thermosetting plastic, a UV-curable resin and the like).

In a particularly preferred form, the flexible straight member is a second flexible tubing portion. Such a method produces two helical portions simultaneously, which can then be separated to provide two separate helical portions. Further, the two helical portions are wrapped around each other, and are thus in intimate contact, which may be advantageous in various situations.

If the pipes are of the same external diameter, then the two helical portions will be identical; however, both helical portions will have a larger amplitude than is envisaged here. Thus, it is preferred for the pipes to have differing diameters, so that the helical portion formed from the larger pipe can have an amplitude which is less than or equal to one half of its internal diameter.

According to a further aspect, there is provided a method of making piping comprising a portion wherein the centreline of the portion follows a substantially helical path, said method including the steps of providing an extruder for extruding a straight pipe, providing a shaping apparatus downstream of said extruder for shaping the extruded pipe into a helical form, and extruding a straight pipe from the extruder and shaping the pipe into a helical form using the shaping apparatus.

This method has the advantage of directly producing a helical portion from raw material, and avoids the need to shape a previously formed straight pipe. It can also produce continuous lengths of helical pipe.

In a preferred form, the shaping apparatus comprises a rotating member, whose axis of rotation is generally parallel to the axis of extrusion, which rotating member has a hole therein through which the pipe passes, the hole being positioned so that its centre is offset from the axis of rotation, the rotating member being driven to rotate as the pipe passes through it to impart a helical shape to the pipe.

Using this shaping apparatus allows the geometry of the pipe to be varied in several ways. For example, the speed of the extruder can be increased or reduced, as can the rotational speed of the rotating member. Further, different rotating members, with the hole in differing positions, can be used.

Preferably, the hole in the rotating member is positioned so that the axis of rotation passes through the hole but is offset from the centre of the hole, so as to produce a helical portion wherein the amplitude of the helix is less than or equal to one half of the internal diameter of the piping and is relatively constant along the portion.

The invention also extends to apparatus for carrying out this method.

According to a further aspect of the invention, there is provided a method of making piping comprising a portion wherein the centreline of the portion follows a substantially helical path, comprising the steps of providing a helical mandrel, winding a flexible pipe around the helical mandrel, so that the pipe assumes a helical geometry, treating the pipe so that it retains its shape, and removing the helical pipe from the mandrel.

This method allows considerable control over the shape of the pipe produced, and also has improved reproducibility when compared to the "twisting" method described above. The geometry of the helical portion is determined by the geometry of the mandrel and the relative sizes of the mandrel and the flexible pipe.

Preferably, the pipe is considerably longer than the helical mandrel, and is wound onto the mandrel at one end thereof, is moved along the helical mandrel and treated so that it retains its shape, and is wound off the mandrel at the other end thereof. This allows the method to be used in a continuous process, rather than a batch process as described above.

It is preferred for the external diameter of the pipe to be greater than the internal diameter of the mandrel, so that the amplitude of the helical pipe produced is less than or equal to one half of the internal diameter of the pipe.

The invention also extends to a helical mandrel for use in the method.

According to a further aspect, there is provided a method of making piping comprising a portion wherein the centreline of the portion follows a substantially helical path, comprising the steps of providing a plurality of short sections of pipe, each having a straight centreline, and having end faces which are not in parallel planes, such that the side has a longest side and a shortest side diametrically opposite to the longest side, connecting two short sections together such that the longest side of one section is slightly rotationally offset from the longest side of the next section, and connecting further short sections, each being slightly rotationally offset from the preceding section by the same amount.

The previous methods are limited to producing pipes of certain materials. In contrast, this method can be used to produce pipes from any suitable material. It is particularly suited to producing metal pipes, which may be required in certain situations (for example, where plastic pipes would be of insufficient strength).

Preferred embodiments of the invention will now be described by way of example only and with reference to the accompanying drawings, in which:

Figure 1 is a view of tubing used in experiments on the flow in a helical portion;
Figure 2 is a view similar to that of Figure 1 but concerning a different experiment;
Figures 3a and 3b illustrate a first method of manufacture of a helical pipe;
Figure 4 illustrates a second method of manufacture of a helical pipe;
Figures 5a to 5e illustrate a third method of manufacture of a helical pipe;
Figures 6a to 6c illustrate a fourth method of manufacture of a helical pipe; and
Figure 7 illustrates the in-plane mixing occurring in and downstream of a helical portion.

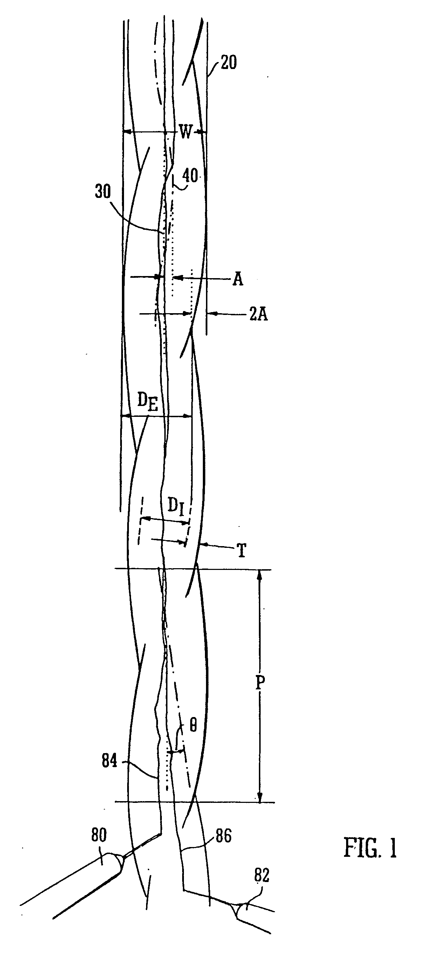

The tubing 10 shown in Figure 1 has a circular cross-section, an external diameter $D_E$, an internal diameter $D_I$ and a wall thickness T. The tubing is coiled into a helix of constant amplitude A (as measured from mean to extreme), constant pitch P, constant helix angle θ and a swept width W. The tubing 10 is contained in an imaginary envelope 20 which extends longitudinally and has a width equal to the swept width W of the helix.
The envelope 20 may be regarded as having a central longitudinal axis 30, which may also be referred to as an axis of helical rotation. The illustrated tubing 10 has a straight axis 30, but it will be appreciated that this axis may instead have a large radius of curvature (either in two or three dimensions). The tubing has a centre line 40 which follows a helical path about the central longitudinal axis 30.

It will be seen that the amplitude A is less than half the tubing internal diameter $D_I$. By keeping the amplitude below this size, the lateral space occupied by the tubing and the overall length of the tubing can be kept relatively small, whilst at the same time the helical configuration of the tubing promotes swirl flow of fluid along the tubing.

A number of experiments were carried out using polyvinyl chloride tubing with a circular cross-section, to establish the characteristics of the flow in a helical portion.

## EXAMPLE 1

Referring to the parameters shown in Figure 1 the tubing had an external diameter $D_E$ of 12mm, an internal diameter $D_I$ of 8mm and a wall thickness T of 2mm. The tubing was coiled into a helix with a pitch P of 45mm and a helix angle θ of 8°. The amplitude A was established by resting the tubing between two straight edges and measuring the space between the straight edges. The amplitude was determined by subtracting the external diameter D. from the swept width W:

$$2A = W - D_E$$

So:

$$A = \frac{W - D_E}{2}$$

In this example the swept width W was 14 mm, so:

$$A = \frac{W - D_E}{2} = \frac{14-12}{2} = 1\ mm$$

As discussed earlier, "relative amplitude" $A_R$ is defined as:

$$A_R = \frac{A}{D_I}$$

In the case of this Example, therefore:

$$A_R = \frac{A}{D_I} = \frac{1}{8} = 0.125$$

Water was passed along the tube. In order to observe the flow characteristics, two needles 80 and 82 passing radially through the tube wall were used to inject visible dye into the flow. The injection sites were near to the central axis 30, i.e. at the "core" of the flow. One needle 80 injected red ink and the other needle 82 blue ink. It will be seen in Figure 1 that the ink filaments 84 and 86 intertwine, indicating that in the core there is swirl flow, i.e. flow which is generally helical. The experiment shown in Figure 1 was carried out at a Reynolds number $R_E$ of 500. In two further experiments, respectively using Reynolds numbers of 250 and 100, swirling core flow was also observed.

## EXAMPLE 2

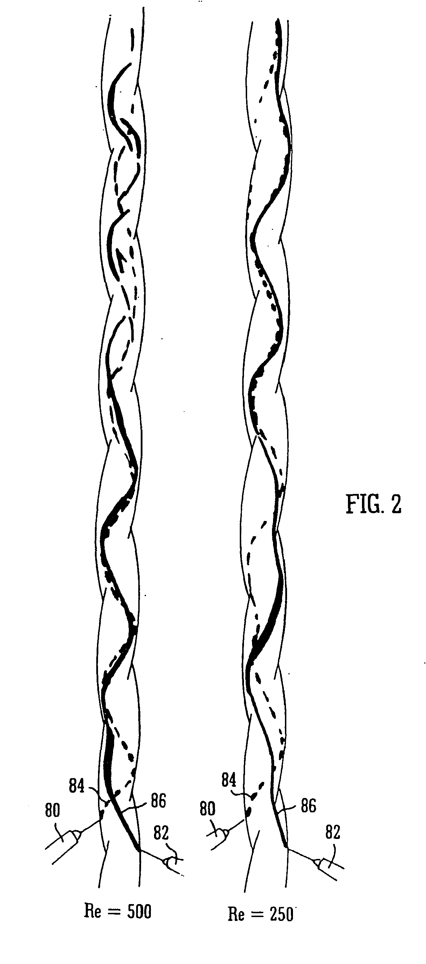

The parameters for this Example were the same as in Example 1, except that the needles 80 and 82 were arranged to release the ink filaments 84 and 86 near to the wall of the tubing. Figure 2 shows the results of two experiments with near-wall ink release, with Reynolds numbers $R_E$ of 500 and 250 respectively. It will be seen that in both cases the ink filaments follow the helical tubing geometry, indicating near-wall swirl.

## EXAMPLE 3

In a separate study, the flow was compared in a straight 8 mm internal diameter tube with that in a helical 8 mm internal diameter tube, where the relative amplitude $A_R$ was 0.45. In both cases the Reynolds number was 500 and 0.2 ml indicator was injected as a bolus through a thin tube at the upstream end. The flows were photographed together with a digital clock to indicate elapsed time after the injection of indicator.

The bolus of indicator, injected into the helical portion, had limited axial dispersion along the pipe, tending to remain coherent. In contrast, in a straight pipe, indicator in the core fluid (near the centre of the pipe) exited the pipe quickly, whereas indicator in fluid near to the walls tended to remain at the walls of the pipe, and took a longer time to exit the pipe. Moreover, the indicator travelled in a more compact mass in the helical tube than in the straight tube. All these findings imply that there was mixing over the tube cross section and blunting of the velocity profile in the helical tube.

## EXAMPLE 4

The experiments of this Example involved a comparison of multi-phase flows in helical tubing with that in tubing having a centreline following a generally sinusoidal path in a single plane. In the case of the helical tubing (whose centre-line curved in three dimensions, i.e. 3D tubing), the internal diameter was 8 mm, the external diameter was 12 mm and the swept width was 17 mm, giving a relative amplitude of 0.3125. The pitch was 90 mm. In the case of the planar, wave-shaped tubing (whose centre-line curved in two dimensions, i.e. 2D tubing), the internal diameter was 8 mm, the external diameter was 12 mm, and the swept width, measured in the plane of the wave shape, was 17 mm. The pitch was 80 mm, not being significantly different from that of the 3D tubing case. The 2D tubing was held with its generally sinusoidal centreline in a vertical plane, in effect creating upwardly convex and concave U-bends.

Both the 3D and 2D tubes were about 400 mm in length, giving 4 to 5 pitches in each case. With both tubes, studies were performed with water flows of 450 and 900 ml per minute (Reynolds numbers of 1200 and 2400 respectively). A needle was used to introduce in all cases a flow of air at a rate of 3 ml per minute, i.e. 0.66% of the water flow in the 450 ml per minute case and 0.33% in the 900 ml per minute case. The air came from a compressed air line and was injected into the tubes just upstream of the start of the respective 3D and 2D geometries.

In the case of the experiment with the 3D tubing at Reynolds number 1200, the air bubbles were about 2 to 3 mm in size and passed along the tube rapidly. At Reynolds number 2400, the bubbles were larger, about 5 to 7 mm but kept moving along the tube with no tendency to stick.

In the case of the 2D tubing at Reynolds numbers of 1200 and 2400, the bubbles were large, about 3 to 5 mm, and tended to stick in the upwardly convex curves (as viewed from outside the tubing).

The experiment shows that in a multi-phase flow the less dense fluid is carried along the 3D tubing, whereas in equivalent 2D tubing the less dense fluid tends to accumulate in the higher parts of the tubing.

As discussed above, when fluid enters a piece of piping shaped as a helical portion in this way, swirl flow is established very quickly. Further, the swirl flow involves considerable secondary motion and mixing of the fluid, with mass transfer between the fluid at the walls of the pipe and the fluid at the centre of the pipe.

This rapid establishment of swirl flow in the helical portion can be used to "condition" the flow, to provide beneficial effects downstream of the helical portion.

As mentioned above, using a pipe having a three-dimensional curve can be better than using a normal (two-dimensional) elbow bend, as the swirl flow established by the three-dimensional curve provides certain benefits. However, it is not normally possible to simply replace an elbow bend by a pipe having a three-dimensional curve; the inlet and outlet pipes of an elbow bend are normally in the same plane, which is not the case with a pipe having a three-dimensional curve. Thus, if a pipe having a three-dimensional curve is to be used in place of an elbow bend, considerable modification can be required to reposition the inlet and/or outlet pipe.

However, the benefits of swirl flow can be achieved with far less modification if a helical portion as described above is fitted upstream of a normal elbow bend. Swirl flow is established rapidly in the helical portion, and this swirl flow continues in the elbow bend.

Since the helical portion has a low amplitude, it can be used in most places where a straight pipe would be used, to "condition" flow in this way to provide the benefits of swirl flow. It should be noted that its use is not limited to elbow bends; it can also be used before T- or Y-junctions, valves, and indeed any form of pipe fitting.

Conditioning the flow in this way is particularly useful before a blind end. Such blind ends can occur at T- or Y-junctions where one of the branches of the junction is closed off (for example, by a valve). With normal flow, the fluid in the part of the branch before the closure tends to stagnate, which can lead to problems with corrosion and the like. However, if the flow is made to swirl before the junction, the swirl extends into the blind end. This prevents stagnation, and avoids the above problems.

A further way of using the helical portions to condition flow is to use them as repeaters. In certain situations, it may not be necessary to provide a continuous length of helical pipe; instead, a straight pipe may have a number of short helical portions arranged along its length. Each portion will induce swirl flow in the fluid passing through it; however, this swirl flow will tend to die away as the fluid passes along the straight pipe. Providing a number of "repeaters" allows the swirl flow to be re-established, with its concomitant benefits.

Helical pipe portions of this type can be made in a number of ways. For example, a straight flexible tube can be wrapped around a straight rigid member (such as a pole), to form it into a helix. The tube can then be removed from the straight rigid member and stretched along the axis of the helix. This stretching has the effect of "flattening out" the helix, in that the pitch is increased and the amplitude is decreased. However, this "flattening out" can distort the helix, and so this method is not preferred.

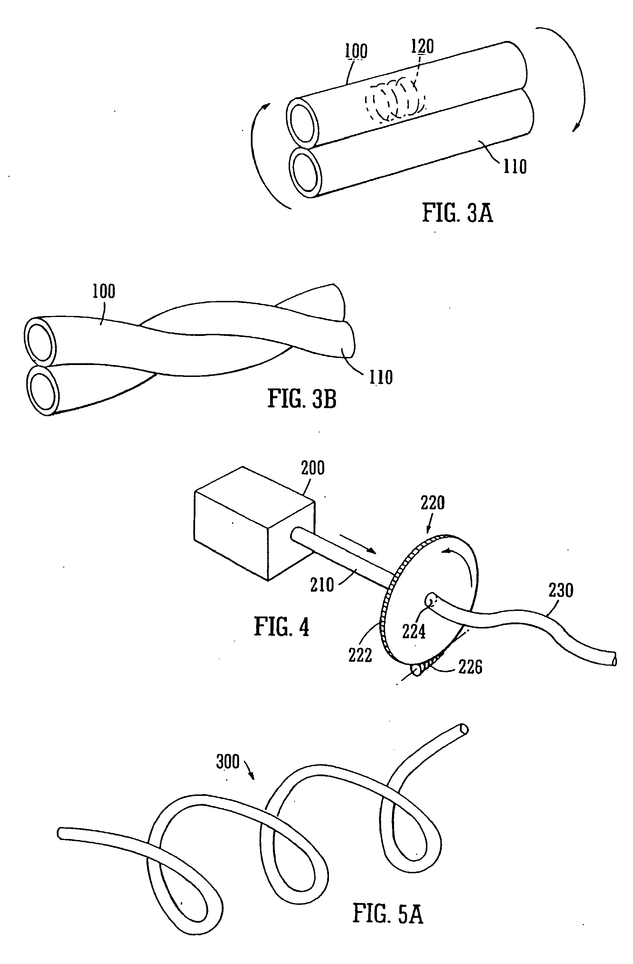

In an alternative method, shown schematically in Figures 3a and 3b, a straight flexible tube 100 is placed next to another straight flexible member 110 (which preferably has a circular cross-section). The ends of the tube and the member are connected to each other, and the assembly is then twisted, which has the effect of making both the tube and the member follow a helical path.

The flexible tube should be prevented from kinking or otherwise deforming in an undesirable manner during twisting. One way of doing this is to insert a snugly fitting coiled spring into the tube before twisting (shown in dotted lines in Figure 3a and denoted by the reference numeral 120).

The flexible tube can be formed from a material which can be made to retain its shape by suitable treatment (for example, a thermosetting plastic, a UV-curable resin and the like). After such treatment, the tube and the member can be removed from each other, to yield a tube formed into a small amplitude helix, which will retain its shape.

In a variant, two such flexible tubes can be laid side by side and have their ends attached to each other; twisting the two tubes then produces two such piping portions, wrapped around each other, which can be separated to produce two separate helical portions.

As an alternative to deforming a straight pipe to produce a helical portion, it is possible to form the helical portion directly during extrusion of the pipe. An apparatus for doing this is shown schematically in Figure 4.

As can be seen, the apparatus includes a conventional pipe extruder 200 which extrudes straight pipes 210. Such extruders are well known, and will not be described further.

Disposed downstream of the outlet of the extruder is an apparatus 220 comprising a rotary member 222, which has a through-hole 224. The through-hole is positioned eccentrically, such that the centre of rotation of the rotary member lies within the through-hole, but does not coincide with the centre of the through-hole. The rotary member is held so that the axis of the through-hole is parallel to the axis of the pipe being extruded, and is driven to rotate. This can be achieved by, for example, teeth on the outer periphery of the rotary member which engage with a worm gear 226, or by any other suitable drive system.

The pipe 210 extruded from the extruder is led through the through-hole 224, and as the pipe is extruded, the rotary member 222 is driven to rotate. As a result of this rotation, the centre of the through-hole is driven to describe a circular path, which in turn forces the pipe being extruded into a helical shape. As the through-hole overlies the centre of rotation of the rotary member, the pipe is formed into a small-amplitude helix 230, as described above.

Once the pipe is shaped into the helix, it can be treated to retain its shape. In practice, the pipe can simply be extruded from a thermoplastic material, and as it cools it will set into the helix shape. This cooling may be achieved using water sprays or similar.

It may be necessary to provide some form of lubrication to ensure that the thermoplastic pipe does not stick in the through-hole. In particular, lubrication may be required to ensure that the pipe does not undergo torsion as it passes through the rotary member.

The particular shape of the helix achieved will depend on several factors, in particular the speed of extrusion, the rate of rotation of the rotary member, and the eccentricity of the through-hole. These can be varied to obtain a particular desired form of helical pipe.

A particularly preferred method of forming a helical portion involves the use of a helical mandrel, and is illustrated in Figures 5a to 5e.

Figure 5a is a schematic illustration of a helical mandrel for use in this method. The mandrel consists of a rigid rod, shaped into a helix. In the embodiment shown, the pitch and the amplitude of the helix are constant along the length of the mandrel, but they may vary.

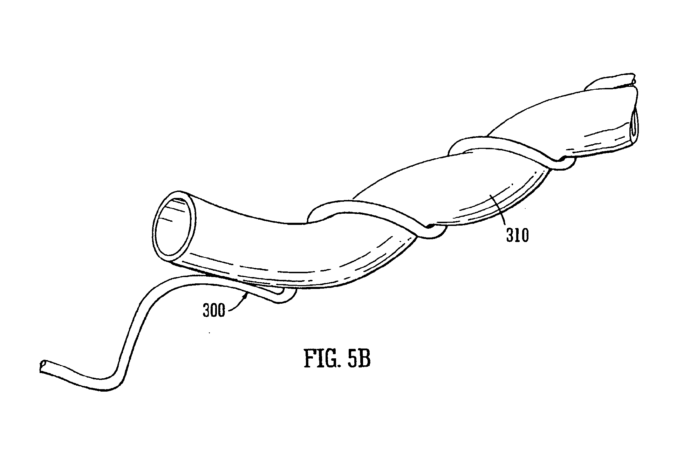

In order to form a helical portion, a length of straight flexible pipe 310, whose external diameter is greater than the internal diameter of the mandrel 300, is wound around the mandrel 300, as shown in Figure 5b. Because the pipe is wider than the space inside the mandrel, it is forced to adopt a helical form, as can be seen from the Figure.

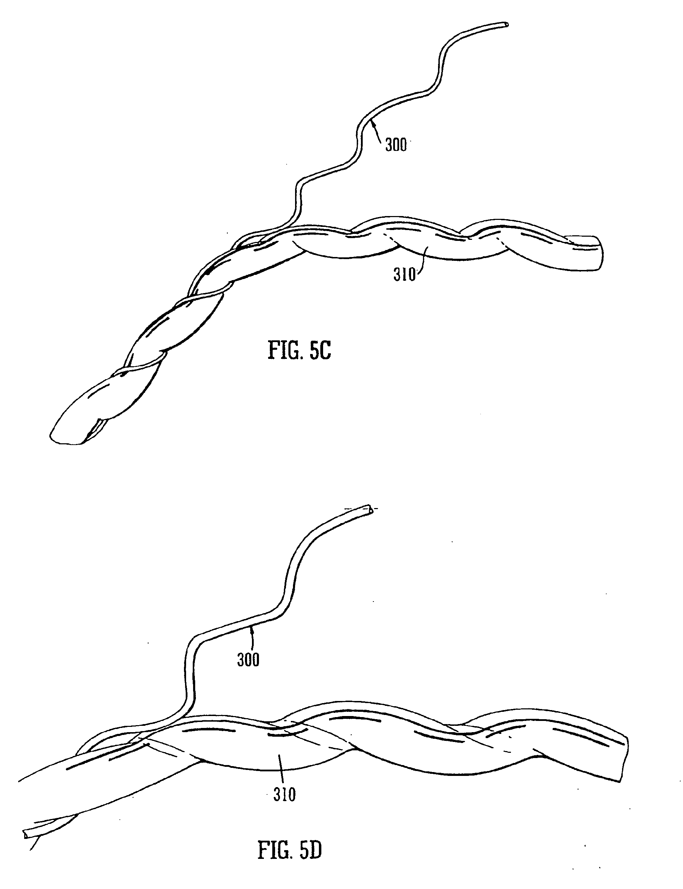

After being treated so that it will retain its helical shape, the pipe can be removed from the mandrel, as shown in Figures 5c and 5d.

As can be seen, the pitch of the helical portion is the same as the pitch of the mandrel. The amplitude of the helical portion will be determined by the diameters of the pipe and of the mandrel.

The above description concerns a batch processing method for forming the helical portion, but this method also lends itself to continuous operation. A continuous length of flexible pipe can be drawn through a comparatively short length of mandrel, and can be treated to retain its shape as it is drawn through (for example, by heating a pipe formed from a thermosetting resin).

Experiment has shown that the pipe rotates relative to the mandrel when it is drawn through in this way. Thus, some form of lubrication may be required to enable smooth functioning of the process. For extremely large pipes and mandrels, it may be desirable to provide roller bearings on the mandrel, rather than lubrication.

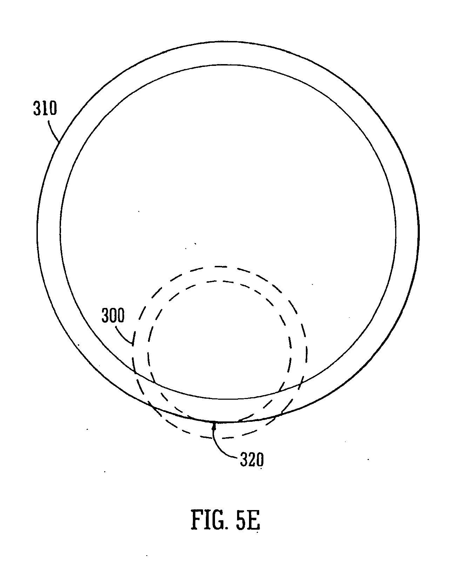

Figure 5e is a schematic cross-section through the pipe 310 and the mandrel 300 as the pipe is drawn. As the helical mandrel is viewed end-on along its axis, it appears as a circle; similarly, the pipe (having a circular cross-section) also appears as a circle in the Figure. It will be seen that the mandrel contacts the outside of the pipe, at point 320, and so the mandrel can be supported from below without interfering with the drawing process.

The mandrel can be formed in any suitable manner, and the method of forming the mandrel will depend to a large extent on the size of the pipes being treated. For relatively small pipes, the mandrel could be formed by winding a rod around a member with a circular cross-section. For larger pipes, the mandrel may need to be machined, for example using a CNC milling machine.

The methods described above are limited to certain materials (such as thermosetting and thermoplastic materials). However, these materials tend to be rather low in strength, and will probably not be suitable for use in more extreme environments, such as offshore, or where very high-pressure fluids must be carried. If a small-amplitude helical pipe is to be used in such situations, then it must be formed in a different manner.

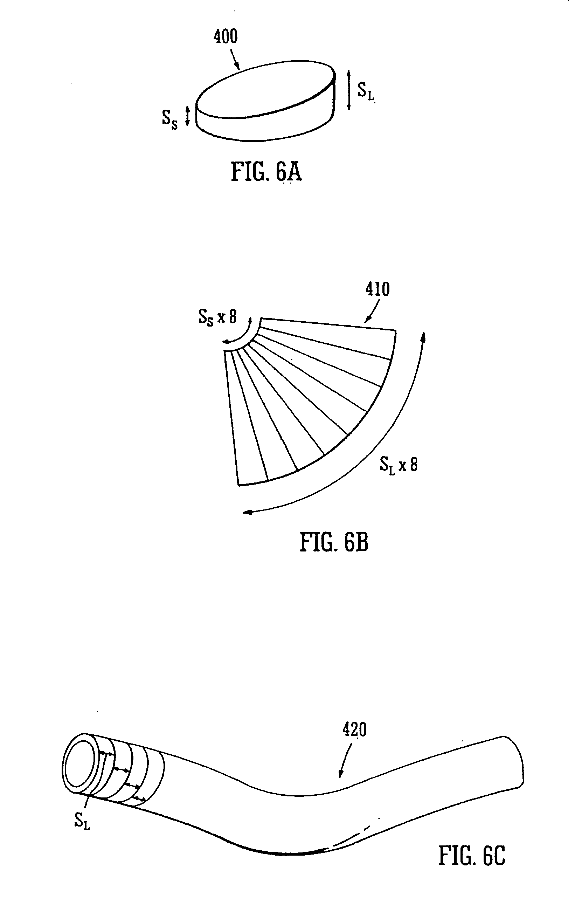

One way of forming a small-amplitude helix for use in high-pressure situations is illustrated with reference to Figures 6a, 6b and 6c.

A known method of forming straight high-pressure pipes is to form them from a large number of short sections, each of which is effectively a very short pipe. Each section has a flange on its upstream and downstream ends, and these flanges co-operate with each other to hold the sections together. In the prior art, the ends of the sections lie in parallel planes, and so when the sections are connected together, the resulting pipe is straight.

However, the segments can also be formed so that their ends lie in planes that are slightly skew. A segment 400 of this type will have one side ($S_L$) which is slightly longer than the diametrically opposite side ($S_9$), as shown in Figure 6a, and can be assembled to form curved pipes, and helical pipes as described above.

To produce a pipe 410 with a two-dimensional curve from short skew-ended pipe sections, the sections are connected so that the longer side of one section connects with the longer side of the previous section, with the shorter sides likewise connecting to each other. As shown in Figure 6b, this produces a pipe with a two-dimensional curve.

To produce a helical pipe 420, the sections are connected together in a similar manner, but each section is slightly rotated relative to the previous section. This is shown in Figure 6c, which shows a helical pipe formed from such sections. At the left-hand of the pipe, the longer sides $S_L$, are shown for the first few sections, and it will be seen that there is relative rotation between the sections. The amount of relative rotation determines the pitch of the helix, with a small relative rotation producing a helix with a small helix angle and a large pitch, and a large relative rotation producing a helix with a large helix angle and a small pitch.

It will be appreciated that at least one end of the pipe will be somewhat elliptical, rather than perfectly circular (as the end is formed by the intersection of a plane cutting a cylinder at an angle to the axis of the cylinder which is not exactly 90°). In a preferred form, both ends are formed so that they are elliptical, as this makes the formation of a two-dimensional curve easier (as the elliptical faces on either end of the segments can match up with each other).

In order to allow the sections to be assembled into a helix, it is necessary for there to be some degree of compliance in the end faces, so that they can accommodate a slight rotation and/or change in shape between the end faces being connected to each other. This can be achieved in any suitable manner, for example by means of an elastomeric material in the end faces.

The effects produced by swirl flow in the helical portion, and in particular the more uniform velocity profile and the improved mixing, can be taken advantage of in a number of situations. In addition, as the overall width of the helical portion is only slightly larger than that of a straight pipe of the same cross-sectional area, the helical portion can be used in virtually any situation where a straight pipe would normally be used.

Helical piping of this type can be used in heat exchangers. These normally take the form of a relatively large-diameter chamber, through which a first fluid flows. In the chamber are mounted a number of small-diameter pipes, through which a second fluid, normally cooler than the first fluid, flows. Heat is exchanged between the two fluids.

Forming the small-diameter pipes from helical piping provides a number of advantages. Firstly, the surface area of a helically-curving pipe is somewhat greater than the surface area of a straight pipe of the same length, and so the available area for heat transfer is increased. More importantly, the improved mixing of fluid in the helically-curving pipe means that fluid at the wall of the pipe, which has been heated by the first fluid, is continually replaced by cooler fluid. This is in contrast to flow in straight pipes, where the fluid at the wall of the pipe tends to stay near the wall. The mixing effect allows all of the fluid in the helically-curving pipe to take part in the heat exchange process, and can improve efficiency.

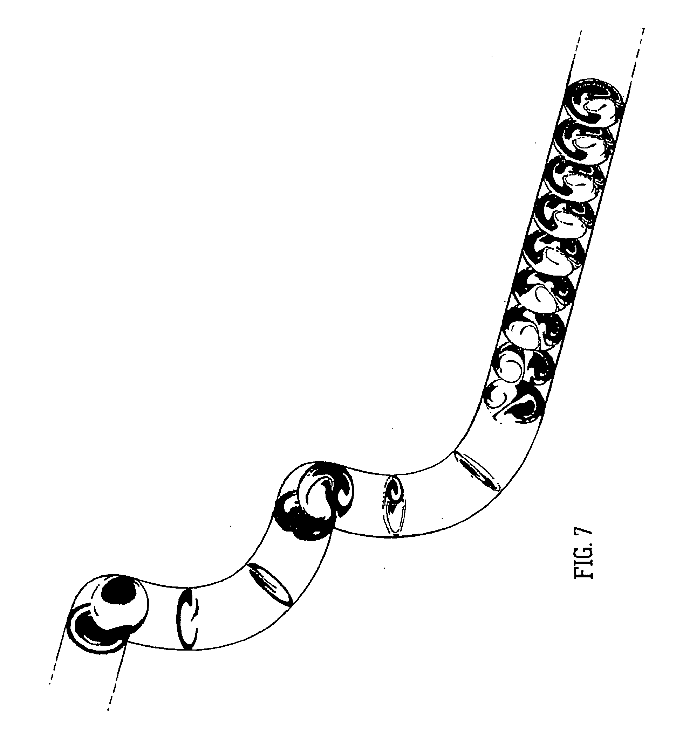

The improved mixing is shown in Figure 7, which shows a helical portion followed by a straight downstream portion. At several points along the portions, the flow is illustrated. The first cross-section of flow is taken on entry to the helical portion; the fluid at the centre of the pipe is represented as darker than the fluid nearer the walls of the pipe. As the fluid moves along the helical portion, it can be seen that there is considerable in-plane mixing in the helical portion, and this mixing continues in the straight portion downstream of the helical portion.

Returning to heat exchangers, it would also be possible to form a heat exchanger from a number of "twisted pairs" of tubes, as described above in the discussion on how to form such tubular portions. Hot fluid would flow in one pipe, and cool fluid in the other. The intimate contact of the tubes allows heat exchange to take place very easily.

A further advantage of swirl flow can be seen in multiphase flow (such as flow of a mixture of a liquid and a gas). Multiphase flow of this type can occur in a great many contexts, such as with liquids close to their boiling points, or in oil drilling, with mixtures of oil and gas. It can also occur with flow of two immiscible fluids of differing densities, such as oil and water, or in a combination of these situations. Multiphase flows can cause a number of problems in conventional pipes, as the gas forms bubbles which, on account of their buoyancy, tend to accumulate in the higher parts of the pipes. If enough gas accumulates, then airlocks can form, seriously affecting the flow. Similarly, with flow of two immiscible liquids, the denser fluid can accumulate in the lower parts of the pipes, causing similar problems.

A further problem with gas accumulation in multiphase flow is that it can lead to "slugging". This phenomenon occurs when gas bubbles collect on the walls of the pipe to such an extent that they block the flow entirely. Fluid approaching this blockage will tend to raise the pressure of the gas, and when the pressure reaches a certain point the blockage will suddenly shift. This "explosion" causes large shock loads on the pipe, and also on any downstream equipment, which can cause serious damage. Indeed, oil production platforms are routinely over-engineered to cope with such loads.

With swirl flow, however, the gas bubbles tend to stay in the centre of the pipe, rather than accumulating at the walls. This is believed to result from the centrifuge effect of the swirl; the denser, liquid part of the flow tends to the walls of the pipe, and the less dense, gaseous part of the flow tends to the centre of the pipe and is entrained by the fluid. There is less chance of a blockage such as an airlock occurring, since the fluids of differing densities have less opportunity to coalesce or pool. There is also far less chance of slugging occurring, as any gas bubbles would be kept away from the wall of the pipe.

Further, as described above, it has been shown experimentally that the bubbles in swirl flow in helical piping tend to be smaller than those in conventional flow in straight pipes. Similar effects would occur with two immiscible liquids of differing densities.

The fact that gas bubbles (or indeed any less dense fraction) tend to the centre of the helical pipe provides further advantages with regard to reduction of the gas content of the flow.

In gas/liquid multiphase flow in a helical pipe, it has been found that the gas occupies a very small cross-sectional area at the centre of the pipe. In comparison to a straight pipe, the concentration of gas across the cross-section is reduced, and this reduction can be up to twenty or thirty percent. (It should be noted that the gas flow rate is the same in both pipes; the flow of the gas is faster in the helical pipe than in the straight pipe, to compensate for the smaller cross-sectional area of flow.)

This reduction in gas concentration can be highly beneficial with, for example, pumps. Pumps are not normally designed to cope with multiphase flow, and do not usually work well with high concentrations of gases. Reducing the concentration of gas in the flow by use of a helical pipe in this way will improve the efficiency of the pump.

A further beneficial effect obtained with multiphase swirl flow is a reduction in pressure drop; reductions of between ten and twenty percent, in comparison to the pressure drop in a straight tube, have been obtained in experiments with vertical pipes. A reduction in pressure drop would also allow an increased flow for the same pressure difference, and so would reduce the amount of energy required to pump a fluid.

The more uniform velocity profile which can be achieved with swirl flow also confers a number of advantages. The flow rate near the wall of the pipe is larger than it is in conventional flow with straight pipes, and so there is less risk of solid material in the pipe being deposited on the wall of the pipe. This is of particular importance if the piping is used to transport slurries or the like.

Dense particulate solids are transported in fluid suspension (ie in a slurry) during a range of mining and extraction processes, and typical flows are 50% solids. In order to avoid the solids settling from the suspension, it is necessary to keep the Reynolds number fairly high. If a straight pipe is used, then it is necessary for the flow velocity to be relatively high, to avoid settling, and this requires more energy to be used in pumping the slurry. However, with helical piping, a reduced flow velocity can be used with no increase in the risk of settling, and so energy consumption can be reduced.

It should be noted that the slurry may be transported significant distances (up to several kilometres), and in order to accommodate the necessary flow rates, the piping may have a diameter of several metres. The beneficial effects of the helical piping can still be achieved in piping of this size.

The increased flow rate near the walls can also inhibit the build-up of biofilms, which can be extremely undesirable. There is also a reduced risk of stagnation regions forming, and since corrosion can occur in stagnant regions, the risk of corrosion is also reduced. These beneficial effects apply to all situations, and not merely to the transport of slurries as described above.

Further, because of the more uniform velocity profile, and the improved mixing between fluid at the wall of the pipe and fluid at the centre of the pipe, the residence time of fluid in the pipe is much more uniform. This is of considerable advantage if the fluid in the pipe is being treated in some way (for example, heated, cooled, irradiated and so on), as the effects of the treatment on the fluid will be more uniform. By way of contrast, in a normal pipe where flow in the centre of the pipe is faster than flow at the walls of the pipe, the residence time will vary (depending on whether the fluid finds itself near the centre or near the wall). Thus, the fluid near the walls will be treated to a greater degree than the fluid in the centre of the pipe, because of its larger residence time. This can be seen from the discussion of Example 3 above.

Another advantage of the secondary motion and mixing associated with swirling flow in a helix is inhibition of the development of flow instability and turbulence; this has been shown experimentally. A further advantage of the more uniform velocity profile is that it reduces axial dispersion and consequent mixing if the same piping is used to transport different materials. This can occur, for example, when a reactor is being filled with ingredients during batch processing.

Axial dispersion is a known problem, particularly with laminar flow, where the fluid at the centre of a pipe flows noticeably faster than the fluid near the walls of a pipe. One way of reducing the axial dispersion is to make the flow turbulent, as this will tend to "flatten" the velocity profile, and make the velocities more uniform across the pipe; however, this can introduce further difficulties, as some fluids (for example, suspensions of macromolecules) can be damaged by the turbulence.

Use of helical portions in the piping allows axial dispersion of batches to be reduced and the peak concentration to be achieved much earlier than with conventional pipes. These features are of particular importance with small batch sizes.

These effects are particularly beneficial in the context of food processing and pharmaceutical production.

Normally in food processing, batches of food are transported through straight pipes. However, because of the velocity profile, the material at the centre of the pipe will tend to move through the pipe at a higher rate than material near the wall of the pipe, and so batches will tend to "spread out" along the pipe. In contrast, if helical piping is used, there is enhanced mixing of material near the centre of the pipe with material near the wall of the pipe, and the batch remains more "coherent". This reduces the change-over time between batches, and also reduces the time necessary to wash the pipe out between batches (as well as reducing the risk of sediment build-up and providing other beneficial effects as described).

In so far as pharmaceutical production also involves the transportation of material along straight pipes, the same beneficial effects can be achieved using helical piping.

The helical portions can also be used in petrochemical processing plant. One particular area where they can be employed is in "crackers". Many cracking processes produce more molecules than are present in the feedstock, and yields rely on a low pressure environment to prevent the molecules from recombining. This is achieved by cooling products in a quench tower, and minimizing pressure loss between the cracking furnace, through the quench tower, to the cracked gas compressor (as yield is inversely proportional to pressure loss). The use of the helical portions in place of straight pipes can reduce the pressure loss, and thus increase yield. Of course, the helical portions can also be used in other areas of petrochemical processing plant.

Further, because of the improved in-plane mixing in the flow, helical pipes of this type can also be used as mixers. A first fluid can be transported in a helical pipe, and a second fluid can be introduced through a branch pipe. The branch pipe can also be a helical pipe, in which case it is desirable for the two pipes to have the same "handedness". This improved mixing, combined with a more uniform residence time, means that the helical pipes can also perform as reactor tubing.

Although specific applications of the helical piping have been described, it will be appreciated that use of the piping is not limited to these applications. Indeed, the piping can be used in any application where the advantages it bestows (more uniform velocity profiles, improved in-plane mixing, reduced axial dispersion, reduced stagnation and so on) would be of benefit.

The following clauses set out features of the invention which may not presently be claimed in this application, but which may form the basis for future amendment or a divisional application.

1. Piping comprising a portion wherein the centreline of the portion follows a substantially helical path, wherein the amplitude of the helix is less than or equal to one half of the internal diameter of the piping.

2. Piping as in clause 1, wherein the portion has a plurality of turns of the helix.

3. Piping as in clause 1 or clause 2, wherein the portion has substantially the same amplitude and helix angle along its length

4. Piping as in any preceding clause, wherein the portion extends along the entire length of the piping.

5. Piping as in any of clauses 1 to 3, wherein the portion only extends along part of the piping.

6. Piping as in any preceding clause, wherein the axis of helical rotation is a straight line.

7. Piping as in any of clauses 1 to 5, wherein the axis of helical rotation is curved.

8. Piping as in any preceding clause, when used as a mixer.

9. A method of making piping comprising a portion wherein the centreline of the portion follows a substantially helical path, said method including the steps of positioning a straight flexible tubing portion adjacent to a further straight flexible member, twisting the flexible tubing portion and the flexible member around each other, and treating the flexible tubing portion so that it retains its shape.

10. A method as in clause 9, wherein the flexible tubing portion is prevented from kinking or otherwise deforming in an undesirable manner during twisting.

11. A method as in clause 10, wherein a snugly fitting coiled spring is inserted into the tubing portion before twisting.

12. A method as in any of clauses 9 to 11, wherein the flexible tubing portion is formed from a material which can be treated so that it retains its shape, such as a thermosetting plastic, a UV-curable resin and the like.

13. A method as in any of clauses 9 to 12, wherein the flexible straight member is a second flexible tubing portion.

14. A method of making piping comprising a portion wherein the centreline of the portion follows a substantially helical path, said method including the steps of:

> providing an extruder for extruding a straight pipe; providing a shaping apparatus downstream of said extruder for shaping the extruded pipe into a helical form; and
> extruding a straight pipe from the extruder and shaping the pipe into a helical form using the shaping apparatus.

15. A method as in clause 14, wherein the shaping apparatus comprises a rotating member, whose axis of rotation is generally parallel to the axis of extrusion, which rotating member has a hole therein through which the pipe passes, the hole being positioned so that its centre is offset from the axis of rotation, the rotating member being driven to rotate as the pipes passes through it to impart a helical shape to the pipe.

16. A method as in clause 15, wherein the hole in the rotating member is positioned so that the axis of rotation passes through the hole but is offset from the centre of the hole, so as to produce a helical portion wherein the amplitude of the helix is less than or equal to one half of the internal diameter of the piping and is relatively constant along the length of the portion.

17. Apparatus for carrying out a method as in any of clauses 14 to 16.

18. A method of making piping comprising a portion wherein the centreline of the portion follows a substantially helical path, comprising the steps of: providing a helical mandrel; winding a flexible pipe around the helical mandrel, so that the pipe assumes a helical geometry; treating the pipe so that it retains its shape; and removing the helical pipe from the mandrel.

19. A method as in clause 18, wherein the pipe is considerably longer than the helical mandrel, and is wound onto the mandrel at one end thereof, is moved along the helical mandrel and treated so that it retains its shape, and is wound off the mandrel at the other end thereof.

20. A method as in clause 18 or clause 19, wherein the external diameter of the pipe is greater than the internal diameter of the mandrel, so that the amplitude of the helical pipe produced is less than or equal to one half of the internal diameter of the pipe.

21. A helical mandrel for use in the method of any of clauses 18 to 20.

22. A method of making piping comprising a portion wherein the centreline of the portion follows a substantially helical path, comprising the steps of: providing a plurality of short sections of pipe, each having a straight centreline, and having end faces which are not in parallel planes, such that the side has a longest side and a shortest side diametrically opposite to the longest side; connecting two short sections together such that the longest side of one section is slightly rotationally offset from the longest side of the next section; and connecting further short sections, each being slightly rotationally offset from the preceding section by the same amount.

## Claims

1.  A method of making piping, and using the piping to carry a multiphase flow comprising a mixture of oil and gas, the piping comprising a portion wherein the centreline of the portion follows a substantially helical path, said method including the steps of positioning a straight flexible tubing portion adjacent to a further straight flexible member, twisting the flexible tubing portion and the flexible member around each other, and treating the flexible tubing portion so that it retains its shape.

**2.** A method as claimed in claim 1, wherein the flexible tubing portion is prevented from kinking or otherwise deforming in an undesirable manner during twisting.

**3.** A method as claimed in claim 2, wherein a snugly fitting coiled spring is inserted into the tubing portion before twisting.

**4.** A method as claimed in any of claims 1 to 3, wherein the flexible tubing portion is formed from a material which can be treated so that it retains its shape, such as a thermosetting plastic, a UV-curable resin and the like.

**5.** A method as claimed in any of claims 1 to 4, wherein the flexible straight member is a second flexible tubing portion.

**6.** A method of making piping, and using the piping to carry a multiphase flow comprising a mixture of oil and gas, the piping comprising a portion wherein the centreline of the portion follows a substantially helical path, said method including the steps of:

provide an extruder for extruding a straight pipe;
providing a shaping apparatus downstream of said extruder for shaping the extruded pipe into a helical form;
and extruding a straight pipe from the extruder and shaping the pipe into a helical form using the shaping apparatus.

**7.** A method as claimed in claim 6, wherein the shaping apparatus comprises a rotating member, whose axis of rotation is generally parallel to the axis of extrusion, which rotating member has a hole therein through which the pipe passes, the hole being positioned so that its centre is offset from the axis of rotation, the rotating member being driven to rotate as the pipe passes through it to impart a helical shape to the pipe.

**8.** A method as claimed in claim 7, wherein the hole in the rotating member is positioned so that the axis of rotation passes through the hole but is offset from the centre of the hole, so as to produce a helical portion wherein the amplitude of the helix is less than or equal to one half of the internal diameter of the piping and is relatively constant along the length of the portion.

**9.** Apparatus for carrying out a method as claimed in any of claims 6 to 8.

**10.** A method of making piping, and using the piping to carry a multiphase flow comprising a mixture of oil and gas, the piping comprising a portion wherein the centreline of the portion follows a substantially helical path, comprising the steps of:

providing a helical mandrel;
winding a flexible pipe around the helical mandrel, so that the pipe assumes a helical geometry;
treating the pipe so that it retains its shape; and
removing the helical pipe from the mandrel.

**11.** A method as claimed in claim 10, wherein the pipe is considerably longer than the helical mandrel, and is wound onto the mandrel at one end thereof, is moved along the helical mandrel and treated so that it retains its shape, and is wound off the mandrel at the other end thereof.

**12.** A method as claimed in claim 10 or claim 11, wherein the external diameter of the pipe is greater than the internal diameter of the mandrel, so that the amplitude of the helical pipe produced is less than or equal to one half of the internal diameter of the pipe.

**13.** A helical mandrel for use in the method of any of claims 10 to 12.

**14.** A method of making piping, and using the piping to carry a multiphase flow comprising a mixture of oil and gas, the piping comprising a portion wherein the centreline of the portion follows a substantially helical path, comprising the steps of:

providing a plurality of short sections of pipe, each having a straight centreline, and having end faces which are not in parallel planes, such that the side has a longest side and a shortest side diametrically opposite to the longest side;
connecting two short sections together such that the longest side of one section is slightly rotationally offset from the longest side of the next section; and
connecting further short sections, each being slightly rotationally offset from the preceding section by the same amount.

FIG. 1

14

EP 2 090 267 A2

FIG. 2

Re = 500        Re = 250

15

FIG. 3A

FIG. 3B

FIG. 4

FIG. 5A

FIG. 5B

FIG. 5C

FIG. 5D

310

300

320

FIG. 5E

400

$S_S$ $S_L$

FIG. 6A

410

$S_S \times 8$

$S_L \times 8$

FIG. 6B

420

$S_L$

FIG. 6C

FIG. 7

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 9728637 A

- WO 02093063 A